# EUROPEAN PATENT SPECIFICATION

(11) **EP 2 741 675 B1**
(45) Date of publication and mention of the grant of the patent: **13.03.2019**
(21) Application number: 12762035.9
(22) Date of filing: 09.08.2012
(51) Int. Cl.: A61B 10/00, C12M 1/30, B01L 3/14, B01L 3/00

(54) **DEVICE FOR THE COLLECTION OF BIOLOGICAL SAMPLES, AND CORRESPONDING METHOD**
VORRICHTUNG ZUR ENTNAHME VON BIOLOGISCHEN PROBEN UND ENTSPRECHENDES VERFAHREN
DISPOSITIF POUR LA COLLECTE D'ÉCHANTILLONS BIOLOGIQUES, ET PROCÉDÉ CORRESPONDANT

(30) Priority: 10.08.2011 IT UD20110129
(43) Date of publication of application: 18.06.2014
(73) Proprietor: Alifax S.r.l., 35020 Polverara (PD) (IT)
(72) Inventor: GALIANO, Paolo, 35100 Padova (IT)
(74) Representative: Petraz, Davide Luigi
(86) International application number: PCT/IB2012/001537
(87) International publication number: WO 2013/021273

(56) References cited:
- EP-A1- 1 346 692
- WO-A2-2009/012307
- US-A1- 2004 057 876
- US-A1- 2011 004 122
- US-A1- 2011 087 133

## Description

### FIELD OF THE INVENTION

The present invention concerns a device usable in laboratory diagnostics, and the corresponding method, for the collection of biological samples, such as pharynx swabs, swabs on wounds and in general skin or mucous swabs, for the replication or growth of bacteria contained in a biological sample in order to detect the presence of said bacteria in the biological sample.

### BACKGROUND OF THE INVENTION

It is known that laboratory diagnostics, with the purpose of detecting the presence of bacteria contained in a biological sample, provides to collect a biological sample and to replicate the bacteria contained therein in order to detect them. Normally, in order to collect said biological samples, one makes use of traditional swabs, of the "cotton bud" type, and new swabs, such as for example that described in the international application WO-A-2004/086979 in the name of Copan Innovation Limited, which provide a solid rod which ends with a point covered in flocculated material formed from hydrophilic fibers which allow the sample taken to be absorbed. This second solution has the disadvantage that it needs to use diluting solutions to remove the bacteria from the swab and additional sowing of the swab in Petri dishes, and therefore does not immediately allow to put the potentially infected sample in contact with a growth broth.

Known swabs are generally used by immersing them in a transport liquid or gel, while cotton buds are used to carry out a dry sampling.

In these state-of-the-art cases, the transport liquid or gel does not provide any assistance to the bacterial replication, and therefore to a subsequent detection, but only allows to maintain the bacteria contained in the sample taken in conditions of neutrality, preventing them from dying.

Instead, solutions using dry sampling lose the bacterial load if the streaking on the Petri dish is carried out some hours after the sample was taken. Document WO-A-2009/012307 provides a device to collect and transport a biological sample in protected conditions which prevent bacterial growth, or mold or fungus growth. The sample, once taken, is put in contact with a swab solution, generally containing preservatives, such as a biocide or in particular an anti-microbial, which allows the stability of the sample, preventing bacterial proliferation. This known device is provided with a container, slidingly separable into two parts, which houses a hollow rod which has a collection swab at one end. In order to take the sample, the separable container is opened, and after having taken the sample using the swab of the hollow rod, the latter is once again housed in the container which is closed, protecting the sample taken. At the upper part, the separable container provides a compressible chamber which contains the swab solution and is selectively in communication with the hollow rod, by means of breaking an upper end segment of the hollow rod. By pressing the chamber, the swab solution is transferred through the hollow rod, toward the collection swab, into contact with the sample taken which is thus transported in protected conditions which prevent bacterial proliferation.

Document US-A-2004/057876 describes a sampling device which provides a compressible collection tube to facilitate the sampling of saliva.

Document US-A-2011/087133 describes some solutions of sampling devices which facilitate reaching internal parts of the body, without any indication relating to the needs of bacterial replication for the purposes of analysis.

Document EP-A-1.346.692 describes a device to collect a biological sample which provides a swab to immerse the sample in a diluting liquid.

Document US-A-2011/004122 describes a collection and drying device of a biological sample for use in forensic medicine, consequently completely unsuitable to collect biological samples and allow the replication of bacteria contained therein for the purposes of diagnostic analysis.

There is therefore a need, in the field of sampling with a standard swab, to develop a device to take a sample of a culture medium and rapidly put it into contact with the bacteria present in the sample, avoiding their early death, which would prejudice the bacterial count of the diagnostic analysis carried out.

One purpose of the present invention is therefore to obtain a collection device and method which use a test tube containing growth liquid consisting of liquid eugenic broth, the purpose of which is to put the bacteria of the sample to be analyzed rapidly into contact with the bacterial culture medium and which facilitate bacterial replication so as to obtain a possible quick growth of the bacteria sampled.

Another purpose of the present invention is to obtain a device and method to introduce the culture broth or growth simply, quickly and safely inside the test tube of the collection device.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a method to take biological samples according to the present invention is intended to replicate bacteria contained in a biological sample in order to detect the presence of said bacteria, and uses at least a test tube able to contain the sample taken and into which a culture broth is introduced, which causes the bacterial replication or growth.

The method provides to use a pick-up unit of the biological sample which is inserted at least partly inside the test tube.

According to the present disclosure the method provides to take in the culture or growth broth into the test tube by means of a suction member of the pick-up unit, determining a stream of culture broth along a hollow rod able to be inserted into the test tube; at one end, the hollow rod is connected fluidically to the suction member and, at an opposite end, it is provided with at least a collection swab for the biological sample which is housed, in the operative step, inside the test tube; the culture broth is made to pass through the collection swab, so as to cause, over all, the culture broth to flow from the suction member toward the inside of the test tube, detaching, in its passage through the collection swab, the bacteria from the collection swab and causing the bacterial replication or growth in the culture broth directly inside the test tube.

The method of the present invention allows the immediate contact of the sample taken with the bacterial growth broth, which facilitates and increases the replication of the bacteria, allowing to obtain an immediate bacterial replication and growth, which can be subsequently detected in the laboratory by an operator qualified to carry out the culture test. This subsequent test can be carried out, for example, by means of streaking on a Petri dish or other techniques, avoiding the disadvantages connected to the loss of bacterial load of dry sampling, and offering an improvement with respect to collecting devices using transport liquid or gel, which are intended only for the neutrality and stability of the bacteria during transport, and not for their replication directly in the collection test tube. Therefore, the present invention offers the analysis laboratory the opportunity to carry out the necessary tests on a biological sample in which a bacterial replication has occurred with respect to the quantity taken, obtaining a thorough bacterial count, for example by means of standard sowing on a Petri dish.

The quick suction stream of the culture broth through the hollow rod and the collection swab has the advantage that it allows to mechanically remove and detach the bacteria possibly present on the swab and thus speed up the bacterial growth in the culture broth, directly in the test tube. Thanks to the suction effect, this detachment and passage allows the immediate contact of the bacteria contained in the sample taken, for example oropharyngeal or nasal mucus, or a swab taken on the skin, with the growth broth in the test tube.

The collection of the material using swabs allows an immediate contact between bacteria and growth liquid, without further decanting of the sample taken. The growth media can be for standard bacteria and for difficult bacteria, such as MRSA or VRSA which use selective growth media.

Unlike what is described in WO-A-2004/086979, the present invention puts the potential infected sample immediately into contact with the growth broth so as to allow an immediate bacterial replication which facilitates the detection both through classic sowing on Petri dishes and other instruments suitable to detect an increased turbidity of the sample, indicating bacterial growth. The liquid growth medium allows a bacterial replication about every 20 minutes and therefore allows the bacterial detection quickly, using suitable instruments for example, such as those used in the light-scattering method.

This immediate contact of the collection swab and the liquid cultural broth allows the clinician to take a safe sample which does not need further opening of stoppers for closing the test tube, as in WO-A-2004/086979, in that the swab inserted in the test tube containing cultural growth broth can be inserted in instruments which automatically perforate the closing stopper of the test tube which contains the sample taken from the swab, already diluted and with an increased quantity of bacteria possibly present at the moment of sampling, for example an oropharyngeal swab or skin swab.

The present invention allows the clinician responsible for carrying out microbiological or bacteriological analyses, taking samples such as oropharyngeal, nasal, or skin swabs, which are immediately immersed in the eugenic growth broth or selective growth media, such as those enriched with antibiotics. Moreover, the present invention allows to carry out the selection of Methicillin resistant bacteria (MRSA) without carrying out further decanting into other specific containers.

The speed of the sampling operations, that is, closing the test tubes and immersing the swab in eugenic growth broth or selected growth media, is an advantage of the present invention in terms of simplicity of execution and offers bacterial replication directly in the sampling test tube.

Moreover, compared with document WO-A-2009/012307, which is intended to prevent any bacterial proliferation thanks to the use of a swab solution containing biocides which is taken in, the present invention allows, once the sample has been taken with the swab, to take in the cultural broth of bacterial growth toward the inside of the test tube, which is rapidly brought into contact with the bacteria or other pathogens contained in the sample taken so that they can be immediately replicated, which facilitates the detection.

The present invention also concerns a device to collect biological samples in a method as set forth above, used for the purpose of replicating bacteria contained in a biological sample to detect the presence of the bacteria. The device of the present invention comprises at least a test tube able to contain the sample taken, into which a culture broth in liquid form which causes the bacterial replication or growth is introduced, and a pick-up unit to take the biological sample, able to be inserted partly inside the test tube.

According to one feature of the present invention, the pick-up unit comprises a first stopper or cap to close the test tube, a suction member associated with one side of the first stopper in order to take in the liquid culture broth, and a hollow rod associated with an opposite side of the first stopper and able to be inserted into the test tube. The hollow rod is connected fluidically at one end to the suction member and at the opposite end is provided with at least a collection swab for the biological sample, able to be housed inside the test tube.

According to the present invention, the collection swab has passage ways able to put the inside of the test tube in communication with the inside of the hollow rod, so as to allow the culture broth taken in to flow through the hollow rod, from the suction member toward the inside of the test tube, detaching, in the passage of the culture broth through the collection swab, the bacteria from the collection swab and causing the bacterial replication or growth in the culture broth directly inside the test tube.

Advantageously, the test tube is made of flexible material able to be deformed so as to determine a depression in order to take in a desired aliquot of culture broth through the tubular suction member.

In accordance with one form of embodiment, the collection swab is made of silicone material, such as molded liquid silicone or silicone paste. In some forms of embodiment the collection swab comprises a shaped body having a coupling portion able to couple with the hollow rod in order to achieve both the mechanical and fluidic connection.

According to a variant, the collection swab, which has a similar function to the traditional cotton bud and can consist of molded liquid silicone or silicone paste, for example with an elliptical section, has a central body comprising a wall with which is associated a chamber delimiting internally a cavity and having an aperture for the mechanical and fluidic connection of the hollow rod by means of the coupling portion. The central body comprises a plurality of transverse hollow pipes for the collection of the biological sample. At least part of the transverse hollow pipes are communicating with the inside of the cavity for the passage of the stream of culture broth taken in, which facilitates the removal of the bacteria present in the collection swab with the passage of the liquid culture broth.

In particular, silicone is a water-repellent material, but, thanks to the stream of culture broth toward the hollow rod, it does not limit the removal of bacteria.

In accordance with another form of embodiment, the collection swab is made of porous sintered material which intrinsically defines internal passage paths so as to obtain the passage ways. The sintered material is also easier to work, to obtain the passage ways of the propagating stream of liquid culture broth.

According to a variant, the collection swab is made of normal absorbent sanitary cotton.

According to another variant, the collection swab comprises a shaped body made of porous sintered material having a coupling pin able to couple with the hollow rod in order to achieve both the mechanical and fluidic connection.

For the forms of embodiment with a molded liquid silicone base or silicone paste, or sintered material, the stream of the broth inserted into the test tube does not vary with respect to the traditional use of material such as cotton buds, and all the different possible materials of which the collection swab is made carry out their sampling function efficiently.

According to some forms of embodiment, the tubular suction member is provided, in a distal position, with a passage hole for the culture broth, associated to a closing cap, which defines, on the first stopper, a longitudinal cavity facing inside the test tube and aligned with said hole, able to determine a mechanical and fluidic connection with the hollow rod connected to the collection swab.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a sectioned perspective view of the device to collect biological samples according to the present invention;
- fig. 2 is a perspective view of a part of the device in fig. 1;
- fig. 3 is a sectioned perspective view of the part in fig. 2;
- fig. 4 is a front view of a variant of the part in fig. 2;
- fig. 5 is a perspective view of the device of the present invention associated to a container of culture broth;
- figs. 6a, 6b, 6c, 6d, 6e are a schematic representation of a sequence of steps for the collection of biological samples using the device in fig. 1.

### DESCRIPTION OF ONE FORM OF EMBODIMENT

With reference to the attached drawings, a device 10 according to the present invention is usable to collect and take biological samples, such as pharynx swabs, swabs from wounds and in general skin or mucous swabs, for the replication of bacteria contained in a biological sample in order to detect the presence of the bacteria in a subsequent bacterial count.

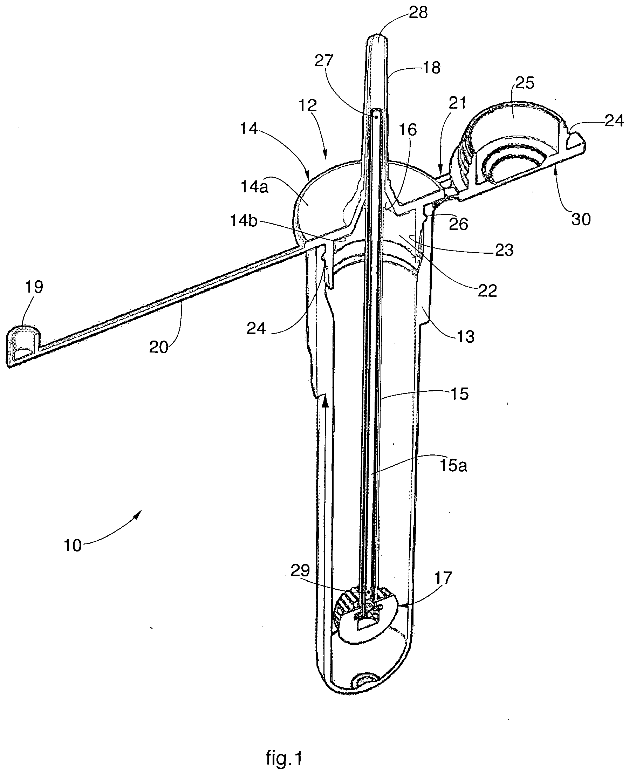

The device 10 (fig. 1) comprises a test tube 13, advantageously made of flexible material so that it can be manually deformed so as to create a desired depression inside it.

The test tube 13 has a shape and size such as to also allow it to be handled by possible analysis devices in the laboratories, without making it necessary to carry out a possible decanting of the biological samples contained therein into subsequent and dedicated test tubes, thus reducing the risk of degrading the quality of the samples themselves.

The device 10 also comprises a pick-up unit 12 to take a biological sample and able to be coupled to, and in part removably inserted into the test tube 13 through an upper aperture 23 thereof.

The pick-up unit 12 comprises a first stopper 14 at the top able to cooperate with the upper aperture 23 of the test tube 13. The first stopper 14 has a tubular suction member 18, in this case of a substantially conical shape, protruding from a first external surface 14a of the first stopper 14. In a distal position, the tubular suction member 18 has a pick-up hole 28 to allow culture or growth broth 32, provided inside a suitable container 36, to be sucked up inside the test tube 13, which causes the replication or growth of the bacteria contained in the sample taken, in order to thus supply an increased quantity of bacteria useful for the bacterial count, for example by means of standard sowing on a Petri dish. Moreover, the tubular suction member 18 can also act as a handle to handle or transport the pick-up unit 12.

Furthermore, on a second internal surface 14b of the first stopper 14, the tubular suction member 18 defines a longitudinal cavity 16 aligned to the hole 28.

The first stopper 14 is provided with a clamping collar 22 which juts out peripherally from the second lower surface 14b of the first stopper 14 and has a shape and size mating with the upper aperture 23 of the test tube 13.

The collar 22 comprises, along its external lateral surface, an annular rib 24 able to make a same-shape coupling with a mating annular groove 26 made along the portion of internal wall of the test tube 13 in correspondence to the aperture 23 of the test tube 13. In this way, the annular rib 24 allows the attachment of the first stopper 14 to the test tube 13 by simple pressure.

The first stopper 14 is associated to a hollow rod or cannula 15, which extends longitudinally from the second internal surface 14b of the first stopper 14.

The hollow rod 15 has a through axial cavity 15a, for the passage of the culture or growth broth 32 toward the inside of the test tube 13.

The hollow rod 15 is also provided with a first end 27, which is able to be coupled inside the longitudinal cavity 16 defined by the tubular suction member 18. In this way, the hollow rod 15 is in fluidic connection with the tubular suction member 18 so that the culture or growth broth 32 can pass from the latter toward the hollow rod 15.

The hollow rod 15 is also provided with a second end 29 able to be inserted inside the test tube 13. The second end 29 carries a collection swab 17, 117 to collect a biological sample which, as will be explained in more detail hereafter, has variously shaped and obtained passage ways, to allow the culture or growth broth 32 to flow from the hollow rod 15 to the inside of the test tube 13 (figs. 1, 2, 3 and 4).

The pick-up unit 12 also comprises a closing cap 19 associable to the hole 28 and which is connected to the first stopper 14 of the pick-up unit 12 by means of a flexible rod 20. The rod 20 has a length and flexibility such that it is able to pass from a first external position to a second position above the tubular suction member 18, in order to allow the positioning of the closing cap 19 above the hole 28 of the tubular suction member 18, so as to close it.

The device 10 also comprises a second stopper 30, able to close the aperture 23 once the pick-up unit 12 is removed. The second stopper 30 is connected, by means of an easily bendable connector 21, to the upper external edge of the test tube 13, in correspondence to the aperture 23.

The second stopper 30, similar in conformation to the first stopper 14, has a collar 25 having an annular rib 31 to cooperate with the annular groove 26 of the test tube 13. The second stopper 30 is used, once the pick-up unit 12 is removed, to close the aperture 23 of the test tube 13 when the biological samples have been inserted into it with the culture or growth broth 32 or equivalent solutions for the growth of bacteria, thus protecting the biological samples acquired from polluting environmental agents.

In figs. 1, 2 and 3 a first form of embodiment of the collection swab is shown, indicated for convenience by the reference number 17.

The collection swab 17, in this case with a morula shape, is advantageously made of silicone because it is easily molded by hot injection, in order to obtain desired shapes and sizes. According to requirements, it is possible to use molded liquid silicone, or silicone paste. The hydrophobic nature of silicone does not affect the outcome of the surface sample-taking because, from comparative tests with other types of known swabs, no substantial differences are found since, with the culture or growth broth 32 used, even a minimum quantity of collected sample is sufficient.

The collection swab 17 has a central body 38 which comprises a wall 43 to which a chamber 45 is associated which delimits internally a cavity 35, through which the culture or growth broth 32, sucked in through the hollow rod 15, is transferred toward the inside of the test tube 13.

In its upper part, the chamber 45 of the central body 38 has an aperture 33 toward the internal cavity 35. Through the aperture 33 the second end 29 of the hollow rod 15 of the pick-up unit 12 is inserted and clamped. The hollow rod 15, by means of a protruding portion 39, in this case of a discoid shape, is removably coupled in an internal annular seating 41 made along the walls of the cavity 35 of the chamber 45.

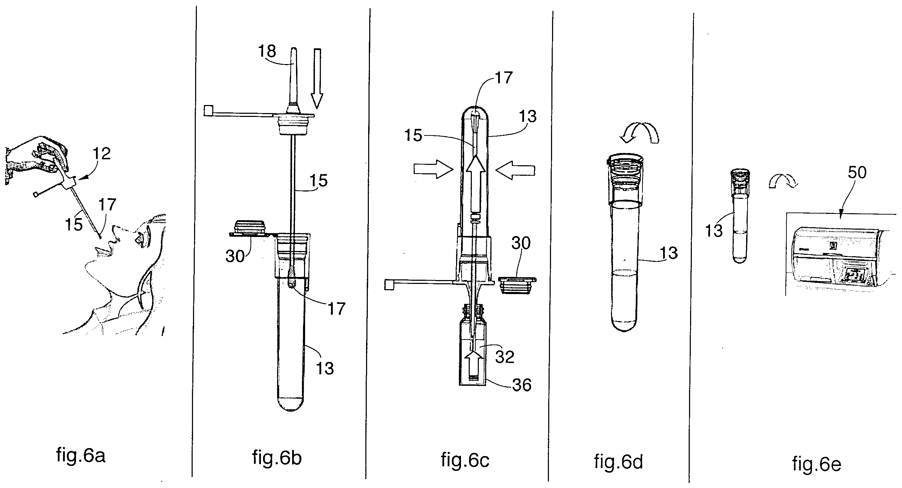

Moreover, the central body 38 supports and positions a plurality of transverse hollow tubes 34 for the collection of the biological sample in the sampling step (fig. 6a), which extend from the wall 43 of the central body 38 and from the chamber 45 toward the outside, on one side and the other.

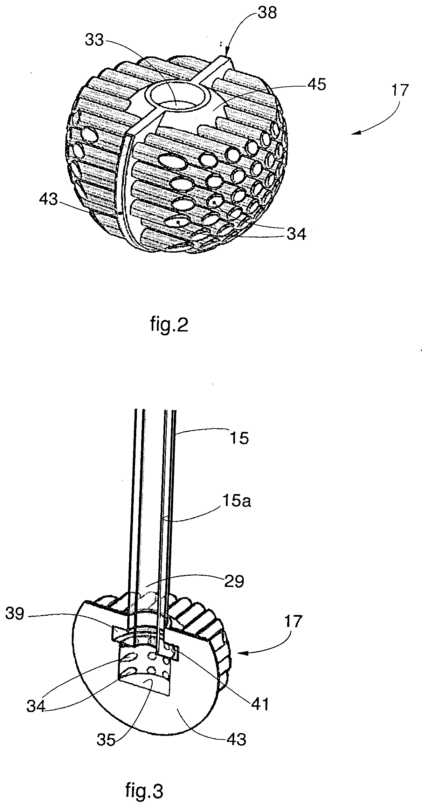

In particular, the transverse hollow tubes 34 provided on the chamber 45 are in communication with the inside of the cavity 35, so as to allow the culture or growth broth 32 to be transferred from the latter to the inside of the test tube 13 (fig. 3).

The transverse hollow tubes 34 are advantageous to facilitate the removal of biological material. Advantageously, the transverse hollow tubes 34 are made of silicone material which, being soft, does not cause discomfort to the patient during the sample taking. The biological tissues taken by the pick-up unit 12 will be contained inside the transverse hollow tubes 34 of the collection swab 17. The transverse hollow tubes 34 are all disposed parallel and are progressively of differentiated lengths, so as to reproduce the desired overall shape of the collection swab 17.

The collection swab 17 can thus have a morula shape as in fig. 2, or a honeycomb or lamellar shape. This guarantees, as we said, also thanks to the intrinsic flexibility of silicone, that a biological sample can be taken painlessly by a normal procedure, such as brushing or grazing the parts from which the sample has to be taken, by the clinician or by the patient himself.

Other variants not shown provide that the collection swab is a compact cotton wad, such as a cotton bud, disposed on the second end 29 of the hollow rod 15. In this case, the cotton's natural propensity for the passage of liquids will promote the transfer of the culture or growth broth 32 from the hollow rod 15 to the inside of the test tube 13.

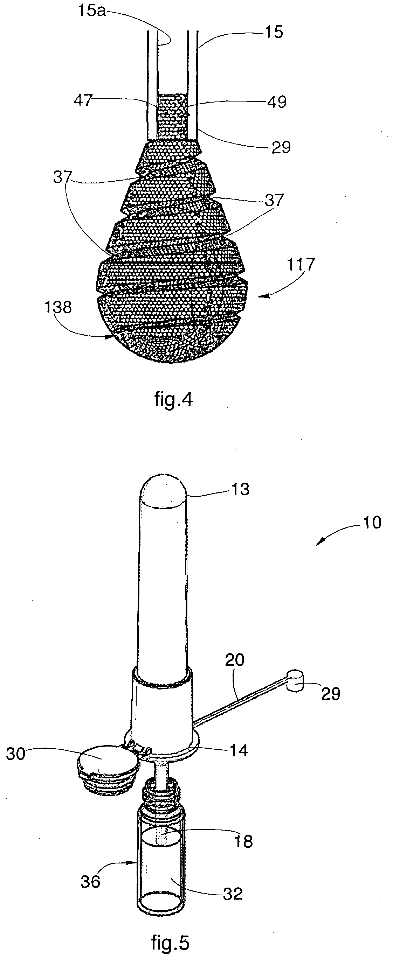

Fig. 4 shows a second form of embodiment of the collection swab, indicated for convenience by the reference number 117. In this case the collection swab 117 is made of porous sintered material which allows an easy release of the bacteria potentially present after the sample has been taken. The collection swab 117 comprises a shaped body 138 made of porous sintered material which, because of its porosity, internally defines a plurality of channels, openings or passage ways for the fluidic collection with the hollow rod 15. In an upper part, the shaped body 138 has a coupling pin 47 made of porous sintered material able to couple with an open attachment seating 49 provided on the second end 29 of the hollow rod 15.

The shaped body 138, in this case, is shaped like a drop, but it can be made in different shapes according to requirements so as to render the sample-taking step as painless, non invasive and efficient as possible.

On the external surface of the shaped body 138 of the collection swab 117, loops or recesses 37 are made with a helical development, with the function of facilitating the acquisition/removal of biological samples during the sample-taking step.

The sintered material of which the collection swab 117 is made, being porous, intrinsically has passage ways for the culture or growth broth 32 coming from inside the hollow rod 15, when sucked through the tubular suction member 18 and, therefore, it allows the diffusion thereof toward the inside of the test tube 13, in this way facilitating the release of the potential bacteria present in the biological samples acquired.

In fig. 5 the device 10 is shown associated to the container 36 of the culture or growth broth 32, once the sample has been taken and the collection swab 17, 117 has been placed inside the test tube 13, closing the latter with the first stopper 14 so as to protect the biological samples from polluting environmental agents. Once the closing cap 19 has been removed, the device 10 is upended, the tubular suction member 18 is immersed into the container 36, so as to dip into the culture or growth broth 32.

The introduction into the test tube 13 of the culture or growth broth 32 thus occurs by suction through the hole 28 of the tubular suction member 18 and from here, through the hollow tube 15 and the corresponding collection swab 17, 117, into the test tube 13; in the passage of the culture or growth broth 32 through the collection swab 17, 117, the bacteria is detached from the latter and is replicated or grows in the culture broth directly in the test tube. The depression which causes the suction effect is obtained by lightly pressing the flexible body of the test tube 13, simply, quickly and without the risk of compromising the quality of the biological samples taken. The culture or growth broth 32 can deposit in the test tube 13 and start the growth and multiplication of the bacteria contained inside the biological sample taken, necessary for subsequent laboratory analysis.

The sample-taking method using the device 10 of the present invention is schematically shown in figs. 6a, 6b, 6c, 6d, 6e and will now be briefly described.

Fig. 6a shows the sample taking step, in this case an oropharyngeal sample, of biological samples by the clinician using the collection swab 17, 117 of the pick-up unit 12. In any case, other zones where samples can be taken are not excluded, such as swiping the skin or other sample-taking methods, for example by means of streaking on Petri dishes.

Fig. 6b shows the insertion step of the pick-up unit 12 into the test tube 13 with the collection swab 17, 117 containing the biological samples taken, taking care to close the aperture 23 with the first stopper 14, thus obtaining a sealed closure and thus guaranteeing the integrity of the samples taken, which do not come into contact with contaminating external agents.

Fig. 6c shows the insertion step of the culture or growth broth 32 inside the test tube 13, without removing or opening the first stopper 14, upending the device 10, introducing the tubular suction member 18 into the container 36 so as to dip into the culture or growth broth 32 and sucking in an aliquot thanks to the depression as described above. Advantageously, the passage of the culture or growth broth 32 also performs a mechanical action to remove the bacteria and biological samples previously taken which, for example, are contained either inside the transverse hollow tubes 34 of the collection swab 17 or on the porous external surface or in the loops 37 of the collection swab 117.

Fig. 6d shows a step in which the pick-up unit 12 is removed from the test tube 13 and, in order to close the aperture 23 of the test tube 13 in a sealed way, the second stopper 30 as above is used.

The test tube 13 thus sealed can be transported to the different laboratories and subsequently placed directly inside the different apparatuses 50 used to carry out the different types of analyses without additional and subsequent decanting (fig. 6e).

## Claims

1. Method for taking biological samples in order to obtain the replication of bacteria contained in a biological sample to detect the presence of said bacteria using at least a test tube (13) able to contain the sample taken and into which a culture or growth broth (32) which causes the replication or growth of said bacteria is introduced, said method providing to use a pick-up unit (12) of the biological sample which is inserted inside said test tube (13), wherein the method provides to take in said culture or growth broth (32) into said test tube (13) by means of a tubular suction member (18) of said pick-up unit (12), determining a stream of said culture or growth broth (32) along a hollow rod (15) able to be inserted into said test tube (13), wherein said hollow rod (15) has an end connected fluidically to said suction member (18) and an opposite end is provided with at least a collection swab (17, 117) for the biological sample, wherein said pick-up unit (18) comprises a first stopper (14) cooperating with an upper aperture (23) of said test tube (13), a step being provided in which the collection swab (17, 117) which carries the biological sample taken is housed inside the test tube (13), closing the latter with the first stopper (14), and the culture or growth broth (32) is made to pass through said collection swab (17, 117), maintaining said test tube (13) closed by said first stopper (14), so as to cause, over all, the stream of said culture broth (32) from said tubular suction member (18) toward the inside of said test tube (13), detaching, in the passage of said culture broth (32) through said collection swab (17, 117), the bacteria from said collection swab (17, 117) and causing the bacterial replication or growth in the culture broth (32) directly inside the test tube (13), and **characterized in that** the method provides using a collection swab (17) having a central body (38) comprising a wall (43) with which is associated a chamber (45) delimiting internally a cavity (35) and having an aperture (33) for the mechanical and fluidic connection of the hollow rod (15), said central body (38) comprising a plurality of transverse hollow pipes (34) for the collection of the biological sample, at least part of said transverse hollow pipes (34) being communicating with the inside of said cavity (35) for the passage of the culture or growth broth (32).

2. Device for the collection of biological samples usable in a method as in claim 1, in order to obtain the replication of bacteria contained in a biological sample to detect the presence of said bacteria, comprising at least a test tube (13) able to contain the sample taken and into which a culture or growth broth (32) which causes the bacterial replication or growth is introduced, and a pick-up unit (12) to take the biological sample, able to be inserted partly inside said test tube (13), wherein said pick-up unit (12) comprises a first stopper (14) to close said test tube (13), a tubular suction member (18) associated with one side of said first stopper (14) in order to take in the culture or growth broth (32), a hollow rod (15) associated with an opposite side of said first stopper (14) and able to be inserted into the test tube (13), said hollow rod (15) being connected fluidically at one end to said tubular suction member (18) and at an opposite end provided with at least a collection swab (17, 117) for the biological sample, able to be housed inside the test tube (13), said collection swab (17, 117) including passage ways able to put the inside of the test tube (13) into communication with the inside of said hollow rod (15), so as to allow the culture or growth broth (32) taken in, when said test tube (13) is closed by said first stopper (14), to flow through said hollow rod (15), from said tubular suction member (18) toward the inside of said test tube (13), detaching, in the passage of said culture broth (32) through said collection swab (17, 117), the bacteria from said collection swab (17, 117) and causing the bacterial replication or growth in the culture broth (32) directly inside the test tube (13), **characterized in that** said collection swab (17) has a central body (38) comprising a wall (43) with which is associated a chamber (45) delimiting internally a cavity (35) and having an aperture (33) for the mechanical and fluidic connection of the hollow rod (15), said central body (38) comprising a plurality of transverse hollow pipes (34) for the collection of the biological sample, at least part of said transverse hollow pipes (34) being communicating with the inside of said cavity (35) for the passage of the culture or growth broth (32).

3. Device as in claim 2, **characterized in that** said test tube (13) is made of flexible and deformable material so as to cause a depression in order to take in a desired aliquot of culture or growth broth (32) through said tubular suction member (18).

4. Device as in claim 2 or 3, **characterized in that** said collection swab (17) is made of silicone material, such as molded liquid silicone or silicone paste.

5. Device as in claim 2 or 3, **characterized in that** said collection swab (17) is made of normal absorbent sanitary cotton.

6. Device as in claim 2 or 3, **characterized in that** said collection swab (117) is made of porous sintered material which intrinsically defines internal passage paths so as to obtain said passage ways.

7. Device as in claim 6, **characterized in that** said collection swab (117) comprises a shaped body (138) made of porous sintered material having a coupling pin (47) able to couple with the hollow rod (15) in order to achieve both the mechanical and fluidic connection.

8. Device as in any claim hereinbefore from 2 to 7, **characterized in that** the tubular suction member (18) is provided, in a distal position, with a passage hole (28) for the culture or growth broth (32) and defines, on the first stopper (14), a longitudinal cavity (16) facing inside the test tube (13) and aligned with said hole (28), able to cause a mechanical and fluidic connection with said hollow rod (15).

## Patentansprüche

1. Verfahren zur Entnahme von biologischen Proben, um das Vermehren von in einer biologischen Probe enthaltenen Bakterien zur erreichen, um die Anwesenheit dieser Bakterien unter Verwendung wenigstens eines Testrohres (13) festzustellen, das dazu eingerichtet ist, die entnommene Probe aufzunehmen und in das eine Kultur- oder Nährlösung (32), die das Vermehren oder ein Wachstum der Bakterien verursacht, eingebracht worden ist, wobei das Verfahren ein Verwenden einer Aufnahmeeinheit (12) für die biologische Probe bereitstellt, die in das Innere des Testrohres (13) eingeführt ist, wobei das Verfahren Aufnehmen der Kultur- oder Nährlösung (32) in das Testrohr (13) mittels eines rohrartigen Saugteils (18) der Aufnahmeeinheit (12) bereitstellt, Bestimmen eines Stroms der Kultur- oder Nährlösung (32) entlang eines hohlen Stabes (15), der dazu eingerichtet ist, in das Testrohr (13) eingeführt zu werden, wobei der hohle Stab (15) über ein Ende verfügt, das fluidmechanisch mit dem Saugteil (18) verbunden ist, und ein gegenüberliegendes Ende mit wenigstens einem Sammeltupfer (17, 117) für die biologische Probe ausgestattet ist, wobei die Aufnahmeeinheit (18) mit einem ersten Anschlag (14) ausgebildet ist, der mit einer oberen Ausnehmung (23) des Testrohres (13) zusammenwirkt, wobei eine Stufe vorhanden ist, in der der Sammeltupfer (17, 117), der die entnommene biologische Probe trägt, innerhalb des Testrohres (13) aufgenommen ist, diesen mit dem ersten Anschlag (14) verschließt und die Kultur- oder Nährlösung (32) dazu bringt, durch den Sammeltupfer (17, 117) unter Beibehalten des Verschlusses des Testrohres (13) durch den ersten Anschlag (14) durchzutreten, so dass insgesamt der Strom der Kulturlösung (32) von dem rohrartigen Saugteil (18) in Richtung der Innenseite des Testrohres (13) bei Durchlauf der Kulturlösung (32) durch den Sammeltupfer (17, 117) die Bakterien von dem Sammeltupfer (17, 117) ablöst und die bakterielle Vermehrung oder das Wachstum in der Kulturlösung (32) direkt innerhalb des Testrohres (13) verursacht, und **dadurch gekennzeichnet, dass** das Verfahren die Verwendung eines Sammeltupfers (17) mit einem eine Wand (43) aufweisenden Zentralkörper (38) bereitstellt, dem eine Kammer (45) zugeordnet ist, die innenseitig einen Hohlraum (35) begrenzt und für die mechanische sowie fluidmechanische Verbindung des hohlen Stabes (15) über eine Ausnehmung (33) verfügt, wobei der Zentralkörper (38) mit einer Anzahl von quer ausgerichteten hohlen Leitungen (34) zum Sammeln der biologischen Probe ausgestattet ist, wobei wenigstens ein Teil der quer ausgerichteten hohlen Leitungen (34) zum Durchlass der Kultur- oder Nährlösung (32) mit der Innenseite des Hohlraums (35) in Verbindung steht.

2. Vorrichtung zum Sammeln von biologischen Proben, die bei einem Verfahren gemäß Anspruch 1 verwendbar ist, um das Vermehren von in einer biologischen Probe enthaltenen Bakterien zu erreichen, um die Anwesenheit dieser Bakterien festzustellen, mit wenigstens einem Testrohr (13), das dazu eingerichtet ist, die entnommene Probe aufzunehmen und in das eine Kultur- oder Nährlösung (32), die das Vermehren oder ein Wachstum der Bakterien verursacht, eingebracht ist, und mit einer Aufnahmeeinheit (12) zum Aufnehmen der biologischen Probe, die dazu eingerichtet ist, teilweise in das Testrohr (13) einführbar zu sein, wobei die Aufnahmeeinheit (12) über einen ersten Anschlag (14) verfügt, um das Testrohr (13) zu verschließen, mit einem rohrartigen Saugteil (18) ausgestattet ist, das einer Seite des ersten Anschlags (14) zugeordnet ist, um die Kultur- oder Nährlösung (32) aufzunehmen, und einen hohlen Stab (15) aufweist, der einer gegenüberliegenden Seite des ersten Anschlags (14) zugeordnet und dazu eingerichtet ist, in das Testrohr (13) einführbar zu sein, wobei der hohle Stab (15) fluidmechanisch mit einem Ende des rohrartigen Saugteils (18) und mit einem an dem wenigstens einen Sammeltupfer (17, 117) für die biologische Probe, die innerhalb des Testrohres (13) aufnehmbar ist, verbindbar ist, wobei der Sammeltupfer (17, 117) Durchlässe aufweist, die dazu eingerichtet sind, die Innenseite des Testrohres (13) mit dem Innern des hohlen Stabes (15) in Verbindung zu setzen, um einer aufgenommenen Kultur- oder Nährlösung (32) bei Verschluss des Testrohres (13) durch den ersten Anschlag (14) ein Strömen durch den hohlen Stab (15) zu gestatten, und zwar von dem rohrartigen Saugteil (18) in Richtung der Innenseite des Testrohres (13), um bei Durchlauf der Kulturlösung (32) durch den Sammeltupfer (17, 117) die Bakterien von dem Sammeltupfer (17, 117) abzulösen und die bakterielle Vermehrung oder das Wachstum in der Kulturlösung (32) direkt innerhalb des Testrohres (13) zu verursachen, **dadurch gekennzeichnet, dass** der Sammeltupfer (17) mit einem eine Wand (43) aufweisenden Zentralkörper (38) ausgebildet ist, die einer innenseitig einen Hohlraum (35) begrenzenden Kammer (45) zugeordnet ist und über eine Ausnehmung (33) für die mechanische sowie fluidmechanische Verbindung des hohlen Stabes (15) verfügt, wobei der Zentralkörper (38) mit einer Anzahl von quer ausgerichteten hohlen Leitungen (34) zum Sammeln der biologischen Probe ausgestattet ist, wobei wenigstens ein Teil der quer ausgerichteten hohlen Leitungen (34) zum Durchlauf der Kultur- oder Nährlösung (32) mit der Innenseite des Hohlraums (35) in Verbindung steht.

3. Vorrichtung nach Anspruch 2, **dadurch gekennzeichnet, dass** das Testrohr (13) aus einem flexiblen und verformbaren Material hergestellt ist, um einen Unterdruck zu verursachen, um die gewünschte Gesamtmenge an Kultur- oder Nährlösung (32) über das rohrartige Saugteil (18) aufzunehmen.

4. Vorrichtung nach Anspruch 2 oder 3, **dadurch gekennzeichnet, dass** der Sammeltupfer (17) aus einem Silikonmaterial, wie einem gespritzten Flüssigsilikon oder Silikonpaste, hergestellt ist.

5. Vorrichtung nach Anspruch 2 oder 3, **dadurch gekennzeichnet, dass** der Sammeltupfer (17) aus üblicher absorbierender Hygienebaumwolle hergestellt ist.

6. Vorrichtung nach Anspruch 2 oder 3, **dadurch gekennzeichnet, dass** der Aufnahmetupfer (117) aus einem porösen gesinterten Material hergestellt ist, das intrinsisch innere Durchlasskanäle bildet, um die Durchlasswege zu bilden.

7. Vorrichtung nach Anspruch 6, **dadurch gekennzeichnet, dass** der Sammeltupfer (117) über einen geformten Körper (138) verfügt, der aus einem porösen gesinterten Material hergestellt ist und der über einen Kopplungsstift (47) verfügt, der dazu eingerichtet ist, mit dem hohlen Stab (15) zu koppeln, um sowohl die mechanische als auch fluidmechanische Verbindung bereitzustellen.

8. Vorrichtung nach einem der Ansprüche 2 bis 7, **dadurch gekennzeichnet, dass** das rohrartige Saugteil (18) in einer distalen Position mit einem Durchlassloch (28) für die Kultur- oder Nährlösung (32) ausgebildet ist und bei dem ersten Anschlag (14) einen länglichen Hohlraum (16) bildet, der in das Innere des Testrohres (13) weist und mit dem Loch (28) ausgerichtet ist, um eine mechanische und fluidmechanische Verbindung mit dem hohlen Stab (15) herbeizuführen.

## Revendications

1. Procédé de prélèvement d'échantillons biologiques en vue d'obtenir la réplication de bactéries contenues dans un échantillon biologique afin de détecter la présence de ces bactéries, en utilisant au moins un tube à essai (13) pouvant contenir l'échantillon prélevé et dans lequel un bouillon de culture ou de croissance (32) qui provoque la réplication ou la croissance desdites bactéries est introduit, ledit procédé prévoyant d'utiliser une unité de collecte (12) de l'échantillon biologique qui est insérée à l'intérieur dudit tube à essai (13), dans lequel le procédé permet de prélever ledit bouillon de culture ou de croissance (32) dans ledit tube à essai (13) au moyen d'un élément d'aspiration tubulaire (18) de ladite unité de collecte (12), en déterminant un flux dudit bouillon de culture ou de croissance (32) le long d'une tige creuse (15) pouvant être insérée dans ledit tube à essai (13), dans lequel ladite tige creuse (15) a une extrémité reliée de manière fluidique audit élément d'aspiration (18) et une extrémité opposée est munie d'au moins un écouvillon de récupération (17, 117) pour l'échantillon biologique, dans lequel ladite unité de collecte (18) comprend un premier bouchon (14) coopérant avec une ouverture supérieure (23) dudit tube à essai (13), une étape étant prévue dans laquelle l'écouvillon de récupération (17, 117) qui porte l'échantillon biologique prélevé est logé à l'intérieur du tube à essai (13), en fermant ce dernier avec le premier bouchon (14), et le bouillon de culture ou de croissance (32) à travers ledit écouvillon de récupération (17, 117), en maintenant ledit tube à essai (13) fermé par ledit premier bouchon (14), de manière à amener, dans l'ensemble, le flux dudit bouillon de culture (32) depuis ledit élément d'aspiration tubulaire (18) vers l'intérieur dudit tube à essai (13), en détachant, lors du passage dudit bouillon de culture (32) à travers ledit écouvillon de récupération (17, 117), les bactéries à partir dudit écouvillon de récupération (17, 117) et en provoquant la réplication ou la croissance bactérienne dans le bouillon de culture (32) directement à l'intérieur du tube à essai (13), et **caractérisé en ce que** le procédé prévoit l'utilisation d'un écouvillon de prélèvement (17) ayant un corps central (38) comprenant une paroi (43) à laquelle est associée une chambre (45) délimitant intérieurement une cavité (35), et comprenant une ouverture (33) une la connexion mécanique et fluidique de la tige creuse (15), ledit corps central (38) comprenant une pluralité de tubes creux transversaux (34) pour la récupération de l'échantillon biologique, au moins une partie desdits tubes creux transversaux (34) communiquant avec l'intérieur de ladite cavité (35) pour le passage du bouillon de culture ou de croissance (32).

2. Dispositif de prélèvement d'échantillons biologiques pouvant être utilisé dans un procédé selon la revendication 1, en vue d'obtenir la réplication de bactéries contenues dans un échantillon biologique afin de détecter la présence de ces bactéries, comprenant au moins un tube à essai (13) pouvant contenir l'échantillon prélevé et dans lequel un bouillon de culture ou de croissance (32) qui provoque la réplication ou la croissance desdites bactéries est introduit, et une unité de collecte (12) pour prélever l'échantillon biologique qui est insérée au moins partiellement à l'intérieur dudit tube à essai (13), dans lequel ladite unité de collecte (12) comprend un premier bouchon (14) pour fermer ledit tube à essai (13), un élément d'aspiration tubulaire (18) associé à un côté dudit premier bouchon (14) afin de prélever le bouillon de culture ou de croissance (32), une tige creuse (15) associée à un côté opposé dudit premier bouchon (14) et pouvant être insérée dans le tube à essai (13), ladite tige creuse (15) étant connectée de manière fluidique à première une extrémité audit élément d'aspiration tubulaire (18) et étant à une extrémité opposée pourvue d'au moins un écouvillon de récupération (17, 117) pour l'échantillon biologique, pouvant être reçu à l'intérieur du tube à essai (13), ledit écouvillon de récupération (17, 117) comprenant des voies de passage capables de mettre l'intérieur du tube à essai (13) en communication avec l'intérieur de ladite tige creuse (15) de manière à permettre au bouillon de culture ou de croissance (32), lorsque ledit tube à essai (13) est fermé par ledit premier bouchon (14), de s'écouler à travers ladite tige creuse (15), à partir dudit élément d'aspiration tubulaire (18) vers l'intérieur du tube à essai (13), en détachant, lors du passage dudit bouillon de culture (32) à travers ledit écouvillon de récupération (17, 117), les bactéries à partir dudit écouvillon de récupération (17, 117), et en provoquant la réplication ou la croissance bactérienne dans le bouillon de culture (32) directement à l'intérieur du tube à essai (13), **caractérisé en ce que** ledit écouvillon de récupération (17) comporte un corps central (38) comprenant une paroi (43) à laquelle est associée une chambre (45) délimitant intérieurement une cavité (35) et présentant une ouverture (33) pour la connexion mécanique et fluidique de la tige creuse (15), ledit corps central (38) comprenant une pluralité de tubes creux transversaux (34) pour la collecte de l'échantillon biologique, au moins une partie desdits tubes creux transversaux (34) étant en communication avec l'intérieur de ladite cavité (35) pour le passage du bouillon de culture ou de croissance (32).

3. Dispositif selon la revendication 2, **caractérisé en ce que** ledit tube à essai (13) est constitué d'un matériau flexible et déformable de manière à provoquer une dépression afin de prélever une aliquote souhaitée de bouillon de culture ou de croissance (32) à travers ledit élément d'aspiration tubulaire (18).

4. Dispositif selon la revendication 2 ou 3, **caractérisé en ce que** ledit écouvillon de récupération (17) est en matériau de silicone, comme une silicone liquide moulée ou une pâte de silicone.

5. Dispositif selon la revendication 2 ou 3, **caractérisé en ce que** ledit écouvillon de récupération (17) est réalisé en coton sanitaire absorbant normal.

6. Dispositif selon la revendication 2 ou 3, **caractérisé en ce que** ledit écouvillon de récupération (117) est constitué d'un matériau fritté poreux qui définit intrinsèquement des trajets de passage internes de manière à obtenir lesdites voies de passage.

7. Dispositif selon la revendication 6, **caractérisé en ce que** ledit écouvillon de récupération (117) comprend un corps mis en forme (138) réalisé en un matériau fritté poreux comportant une broche de couplage (47) apte à se coupler à la tige creuse (15) afin de réaliser la connexion mécanique et fluidique.

8. Dispositif selon l'une quelconque des revendications 2 à 7 ci-dessus, **caractérisé en ce que** l'élément d'aspiration tubulaire (18) est pourvu, dans une position distale, d'un trou de passage (28) pour le bouillon de culture ou de croissance (32) et définit, sur le premier bouchon (14), une cavité longitudinale (16) orientée vers l'intérieur du tube à essai (13) et alignée avec ledit trou (28), capable de provoquer une connexion mécanique et fluidique avec ladite tige creuse (15).
